(19)

Europäisches Patentamt
European Patent Office
Office européen des brevets

(11) **EP 3 970 620 A1**

(12) **EUROPEAN PATENT APPLICATION**

(43) Date of publication:
**23.03.2022  Bulletin 2022/12**

(21) Application number: **20197184.3**

(22) Date of filing: **21.09.2020**

(51) International Patent Classification (IPC):
***A61B 6/00*** *(2006.01)*

(52) Cooperative Patent Classification (CPC):
**A61B 6/483; A61B 6/48; A61B 6/484; A61B 6/50;
A61B 6/5205; A61B 6/5217; A61G 13/04**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **VON BERG, Jorn**
  **5656 AE Eindhoven (NL)**
• **KOEHLER, Thomas**
  **5656 AE Eindhoven (NL)**

(74) Representative: **Philips Intellectual Property &
Standards
High Tech Campus 52
5656 AG Eindhoven (NL)**

(54) **PROCESSING DARK-FIELD X-RAY IMAGE DATA INFORMATION**

(57)    Provided is an apparatus (10) for processing dark-field X-ray image data information. The apparatus comprises a data interface (20); and a data processing unit (30). The data interface (20) is configured to provide the data processing unit with dark-field X-ray image data. The data processing unit (30) is configured to segment the image data to provide segmented image data of the lung, the segmented image data comprising at least a first lung segment and a second lung segment distinguishable therefrom, the first lung segment and the second lung segment being segmented to have a defined volume. The data processing unit (30) is further configured to derive, from the image data, at least a first dark-field signal value assigned to the first lung segment and a second dark-field signal value assigned to the second lung segment. Further, the data processing unit (30) is configured to derive a quantitative measure by comparing the first dark-field signal value and the second dark-field signal value with each other, the quantitative measure being output data for lung analysis.

Fig. 1

EP 3 970 620 A1

## Description

FIELD OF THE INVENTION

The present invention relates to dark-field X-ray imaging, and in particular to an apparatus for processing dark-field X-ray image data, a method for processing dark-field X-ray image data, an X-ray imaging system, a computer program element, and a computer readable medium.

BACKGROUND OF THE INVENTION

In medical applications, such as lung analysis etc., there may be some impairments, disorders, abnormalities, or the like, which can be detected at least easier or more reliably by utilizing some imaging techniques than by others. For example, some impairments etc. of a subject can be detected more reliably with computed tomography (CT), while other impairments cannot be detected reliably or at all with CT.

In this regard, X-ray dark field radiography is a promising imaging technology for the detection of particularly pulmonary disorders. However, it is unclear how image data derived from X-ray dark field radiography may be processed to obtain further information, particularly useful for lung analysis or for diagnostic purposes.

SUMMARY OF THE INVENTION

There may, therefore, be a need for improved means for processing dark-field image data. The object of the present invention is solved by the subject matter of the independent claims, wherein further embodiments are incorporated in the dependent claims.

According to a first aspect, there is provided an apparatus for processing X-ray image data, preferably dark-field X-ray image data. The apparatus comprises at least one data interface; and at least one data processing unit. The data interface is configured to provide the data processing unit with dark-field X-ray image data. The data processing unit is configured to segment the image data to provide segmented image data of the lung, the segmented image data comprising at least two lung segments, namely at least a first lung segment and a second lung segment, distinguishable therefrom, the first lung segment and the second lung segment being segmented each to have a defined volume. The data processing unit is configured to derive, from the image data itself and/or from further calculations, parameters etc., at least a first dark-field signal value assigned to the first lung segment and a second dark-field signal value assigned to the second lung segment. The data processing unit is configured to derive a quantitative measure by comparing the first dark-field signal value and the second dark-field signal value with each other, the quantitative measure being output data for lung analysis.

In this way, an at least semi-automatic means for deriving the quantitative measure from the image data is provided, the quantitative measure forming at least a data basis for further lung analysis or diagnosis. The apparatus provided may allow to differentiate image data of subjects with lung impairment from image data of inconspicuous subjects with no or less lung impairment. In this regard, it has been found that some pulmonary impairments, disorders, abnormalities, or the like, of a subject can be detected easier and/or more reliably by utilizing dark-field X-ray imaging techniques, and particularly if the lung recorded in the image data is firstly segmented into two or more segments and then the respective dark-field signal values assigned to the respective lung segment are evaluated with respect to the lung segment volumes. By way of example, the apparatus according to the first aspect may be particularly suitable and/or sensitive to detect emphysema, COPD, etc., in a more reliable manner, or to detect emphysema already in an early stage, where it cannot be detected or reliably detected by utilizing other imaging techniques, such as CT. It is also recognized in this disclosure that the specific selection of lung segments with respect to their location relative to each other, and/or relative to the anatomy of the subject, may also be a factor to be considered in order to be able to derive the quantitative measure for lung analysis and/or diagnosis.

In this description, two segments are designated or referred to. It is to be understood, however, that although at least two segments are formed, the number of segments is not limited to two, but multiple segments, that is, a plurality of segments, may be formed. For example, left lung and right lung may be separated first, and then each lung, i.e. the left lung and/or the right lung, may be segmented in two or more segments.

As used herein, segmentation of image data may be understood as a computer-implemented process of partitioning a digital image into multiple segments to simplify and/or change the representation of the image. The segmented image data derived from this segmentation process may be more meaningful to a reader and/or easier to analyze. For example, the lung captured in the digital image may be partitioned into two or more segments, which may also be referred to as sets of image objects and/or sets of pixels, separated from each other. Thereby, the segmentation process may, for example, utilize that pixels within a region are similar with respect to some characteristic or computed property, such as color, intensity, or texture, while adjacent regions are significantly different with respect to the same characteristic. There are several segmentation methods that can be utilized in this context, such as clustering methods, e.g. by means of a K-means algorithm, histogram-based methods, the use of a convolutional neural network trained for this purpose, etc.

The defined volume of the lung segments as used herein may be understood in a way that the volume of the respective segment is known, e.g. from the segmentation process or at least can be determined therefrom and/or the image data, preferably in a numerical value, a likelihood, or the like.

The quantitative measure as used herein may, for example, be understood as an indicator for the possible presence of an impairment, abnormality or the like of the lung recorded in the image data. For example, the quantitative measure may be a meaningful value, such as a numerical value or the like. By way of example, the quantitative measure may comprise one or more of a difference in the dark-field signal values between the lung segments, a standard deviation of the dark-field signal values over all segments and/or areas, etc., of the lung, a normalized standard deviation of the dark-field signal values over all segments and/or areas, etc., of the lung, and/or a ratio of the dark-field signal values of the lung segments. The quantitative measure may then be provided as output data for (further) lung analysis, which can also be part of a subsequent process, which preferably is carried out ex-vivo. On the basis of the quantitative measure, the lung analysis may be carried out either at least semi-automatically by utilizing computer means or manually by a medical professional, such as a radiologist etc.

Each lung segment may have a dark-field signal value, e.g. a signal strength, wherein the respective dark-field signal values of each lung segment may be compared to and/or evaluated with respect to the other lung segment(s).

As used herein, the X-ray image data may refer to at least one attenuation image in which the intensity of the image is proportional to the integral of the transmission properties (determined by density, material permittivity, etc.) of tissue through which the X-ray has propagated, so that an X-ray attenuation image may be a classical X-ray image, and/or may refer to X-ray information containing a scattering component of an applied X-ray. The latter may be obtained using an interferometric detection approach, wherein other approaches are known. Microstructures in lung may cause scattering of an X-ray wave, wherein the scattering component has been integrated into a final X-ray attenuation value, and hence has not been separable. Using interferometric X-ray imaging techniques, for example, it is possible to distinguish the scatter component of the X-ray signal from the attenuation and phase components, to provide extra information about the internal structure of the lung.

According to an embodiment, the first lung segment and the second lung segment may be segmented to have at least approximately equal volumes. In other words, the at least two lung segments may have, after performing the segmentation process, at least substantially equal or the same volumes. In this way, e.g. through a normalization of the dark field signal with respect to the lung segment volume, the effect of the size of the object can be mitigated. For example, by normalizing the dark field signal, material constant information for the lung can be determined that is de-convolved from the length through the lung, enabling healthy lung tissue to be differentiated from pathological tissue. In this way, by forming lung segments having the same volume, the dark-field signal values referring to the respective lung segment can be directly evaluated, i.e. compared with each other.

In an embodiment, comparing the first dark-field signal value and the second dark-field signal value may comprise determining a ratio from the at least two values to derive the quantitative measure. For example, it has been found that subjects having a lung impairment, e.g. an emphysema or the like, typically have a ratio that is different to that of inconspicuous subjects with no or less lung impairment. By way of example, subjects having an emphysema and/or COPD tend to have a specific ratio of the dark-field signal values of the upper lung and lower lung, so that the ratio allows a differentiation to subjects without lung impairment. In particular, emphysema and/or COPD subjects may have a higher upper-to-lower-lung field-ratio. That is, in this embodiment, the lung or lung field may be segmented into two segments, wherein a first segment is assigned to the upper lung and a second segment is assigned to the lower lung, and wherein the terms upper and lower refer to a longitudinal axis of a subject's body. Based on this differentiation between upper and lower part of the lung, the dark-field signal may be determined, calculated etc. individually for each part.

According to an embodiment, the ratio may be an indicator of at least the presence, optionally with a likelihood assigned thereto, of a disease or finding in the lungs. For example, the ratio may allow an emphysema to be detected.

In an embodiment, the first lung segment may be an upper lung field and the second lung segment may be a lower lung field, the lung fields defined as upper and lower with respect to a longitudinal axis of a subject's body. As mentioned above, subjects having e.g. an emphysema tend to have a specific ratio of the dark-field signal values of the upper lung and lower lung, so that segmenting the upper and lower lung fields allows a differentiation to subjects without lung impairment.

According to an embodiment, the data processing unit may be further configured to estimate a lung depth, the lung depth defined along a normal of a projection plane of the image data, for a plurality of different positions within the corresponding lung segment. The plurality of different positions may correspond to or may also be referred to a plurality of, particularly different, pixels of an image included in the image data. In this way, the segments may be determined so that they have a defined, i.e. a certain, volume and this volume is known, or they may be determined so that they have at least approximately the same volume. The volume may be calculated based on an area of the lung segment that may be determined from the image data.

In an embodiment, the data processing unit is further configured to determine the corresponding segment a volume on the basis of a lung depth determination, the lung depth defined along a normal of a projection plane of the image data, and an area determination from the image data to obtain the defined segment volumes.

According to an embodiment, the different positions are assigned to different pixels of an acquired image comprised in the image data. In this way, segments can be formed with pixel accuracy.

In an embodiment, the data processing unit is further configured to normalize the corresponding dark-field signal value to a lung depth, the lung depth defined along a normal of a projection plane of the image data. In this way, dark field signals can be normalized to the lung depth.

According to an embodiment, the data processing unit is further configured to determine the corresponding dark-field signal value of the segment volume on the basis of a lung depth determination, the lung depth defined along a normal of a projection plane of the image data. In this way, dark field signals can be normalized to the lung volume.

In an embodiment, the data processing unit is further configured to separate a left lung and a right lung to provide the segmented image data of the lung, wherein for each lung the first lung segment and the second lung segment is segmented. In other words, in a first step, the left and right lung may be separated from each other, and, in a second step, the left and/or right lung is segmented as described above.

According to a second aspect, there is provided an X-ray imaging system. The system comprises an image acquisition device that is configured to acquire X-ray image data of a subject, and the apparatus according to the first aspect.

According to a third aspect, there is provided a method for processing X-ray image data, preferably dark-field X-ray image data. The method comprises the steps of receiving, by a data processing unit, dark-field X-ray image data of a subject's lung, segmenting, by the data processing unit, the image data to provide segmented image data of the lung, the segmented image data comprising at least a first lung segment and a second lung segment distinguishable therefrom, the first lung segment and the second lung segment being segmented each to have a defined volume, deriving, by the data processing unit, from the image data at least a first dark-field signal value assigned to the first lung segment and a second dark-field signal value assigned to the second lung segment; and deriving, by the data processing unit, a quantitative measure by comparing the first dark-field signal value and the second dark-field signal value with each other, the quantitative measure being output data for lung analysis.

Preferably, the method is computer-implemented. For example, the method may be carried out by means of the apparatus according to the first aspect, and/or by means of the system according to the second aspect.

According to a fourth aspect, there is provided a computer program element, which when executed by a processor is configured to carry out the method of the third aspect, and/or to control a system according to the second aspect, and/or to control an apparatus according to the first aspect.

According to a fifth aspect, there is provided a computer-readable storage or transmission medium, which has stored or which carries the computer program element according to the fourth aspect.

It is noted that the above embodiments may be combined with each other irrespective of the aspect involved. Accordingly, the method may be combined with structural features of the apparatus and/or system of the other aspects and, likewise, the apparatus and/or the system may be combined with features of each other, and may also be combined with features described above with regard to the method. Advantageously, the benefits provided by any of the above aspects and examples equally apply to all of the other aspects and examples and vice versa.

These and other aspects of the present invention will become apparent from and elucidated with reference to the embodiments described hereinafter.

BRIEF DESCRIPTION OF THE DRAWINGS

Exemplary embodiments of this disclosure will be described in the following drawings.

Fig. 1 shows an example of an apparatus for processing dark field X-ray image data according to an embodiment.
Fig. 2 shows an example of a system for processing dark field X-ray image data according to an embodiment.
Fig. 3 shows in a flow chart an example of a method for processing dark-field X-ray image data according to an embodiment.
Fig. 4 shows an example of a segmentation of a lung recorded in image data according to an embodiment.
Fig. 5 shows another example of a segmentation of a lung recorded in image data according to an embodiment.
Fig. 6 illustrates in a chart how a ratio of dark-field signals can be used in processing dark-field X-ray image data according to an embodiment.

DETAILED DESCRIPTION OF EMBODIMENTS

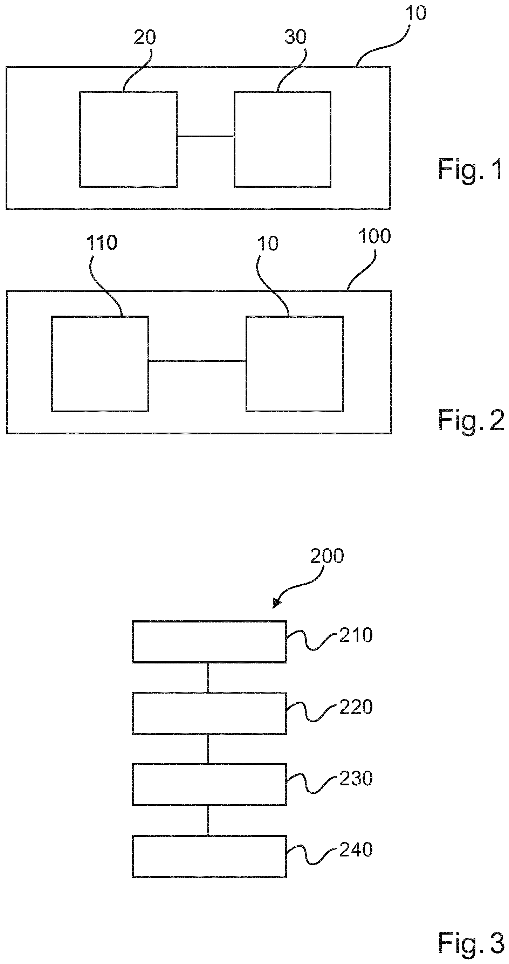

Fig. 1 shows an example of an apparatus 10 for processing dark-field X-ray image data. The apparatus 10 comprises a data interface 20 and a data processing unit 30. The data interface 20 may comprise means for data input and/or data output. For example, the data interface 20 may be configured to receive data, e.g. image data, from an image acquisition device 110 (see Fig. 2) and to provide the data received to the data processing unit 30, and/or to receive data from the data processing unit 30 and to provide the data received to a further entity for e.g. representation of the data processed, further lung analysis, etc. The image data received may comprise X-ray attenuation information of a patient's chest. The data processing unit 30 is further configured to segment the image data to provide segmented image data of the lung. The segmented image data comprises at least a first lung segment and a second lung segment, wherein the segments are distinguishable from each other. The first lung segment and the second lung segment are segmented to each have a defined volume V, i.e. a volume known. The data processing unit 30 is configured to derive, from the image data, at least a first dark-field signal value DAX and/or NDAX assigned to the first lung segment and a second dark-field signal value assigned to the second lung segment DAX and/or NDAX. Further, the data processing unit 30 is configured to derive a quantitative measure by comparing the first dark-field signal value DAX and/or NDAX and the second dark-field signal value DAX and/or NDAX with each other. The quantitative measure may then be provided as output data for a further lung analysis, diagnosis, or the like.

In an example, the first lung segment and the second lung segment may be segmented to have at least approximately equal volumes. In other words, the at least two lung segments may have, after performing the segmentation process, at least substantially equal or the same volumes V. In this way, through a normalization of the dark field signal with respect to the corresponding lung segment volume V, the effect of the size of the object can be mitigated. For example, by normalizing the dark field signal DAX and/or NDAX, material constant information for the lung can be determined that is de-convolved from the length through the lung, enabling healthy lung tissue to be differentiated from pathological tissue. In this way, by forming lung segments having the same volume, the dark-field signal values referring to the respective lung segment can be directly evaluated, i.e. compared with each other.

In an example, the first dark-field signal value DAX and/or NDAX and the second dark-field signal value DAX and/or NDAX may be evaluated, e.g. compared with each other, wherein the evaluation comprises determining a ratio from the at least two values to derive the quantitative measure. For example, it has been found that subjects having a lung impairment, e.g. an emphysema and/or COPD or the like, typically have a ratio that is different to that of inconspicuous subjects with no or less lung impairment. By way of example, subjects having an emphysema tend to have a specific ratio of the dark-field signal values of the upper lung and lower lung, so that the ratio allows a differentiation to subjects without lung impairment. In particular, e.g. emphysema subjects may have a higher upper-to-lower-lung field-ratio. That is, in this example, the lung or lung field may be segmented into two segments, wherein a first segment is assigned to the upper lung and a second segment is assigned to the lower lung, and wherein the terms upper and lower refer to a longitudinal axis of a subject's body. Based on this differentiation between upper and lower part of the lung, the dark-field signal may be determined, calculated etc. individually for each part. In an example, the ratio may be an indicator of at least the presence, optionally with a likelihood assigned thereto, of a disease or finding in the lungs. For example, the ratio may allow an emphysema to be detected.

In an example, the first lung segment may be an upper lung field and the second lung segment may be a lower lung field, the lung fields defined as upper and lower with respect to the longitudinal axis of a subject's body. As mentioned above, subjects having e.g. an emphysema tend to have a specific ratio of the dark-field signal values of the upper lung and lower lung, so that segmenting the upper and lower lung fields allows a differentiation to subjects without lung impairment.

In an example, the data processing unit 30 may be further configured to estimate a lung depth, the lung depth defined along a normal of a projection plane of the image data, for a plurality of different positions within the corresponding lung segment. The lung depth may be estimated using an image processing method that achieves signal separation after acquisition of the X-ray attenuation and X-ray dark-field image information. The plurality of different positions may correspond to or may also be referred to a plurality of, particularly different, pixels of an image included in the image data. In this way, the segments may be determined so that they have a defined, i.e. a certain, volume and this volume is known, or they may be determined so that they have at least approximately the same volume. The volume may be calculated based on an area of the lung segment that may be determined from the image data.

In an example, the data processing unit 30 is further configured to determine the corresponding segment a volume on the basis of a lung depth determination, the lung depth defined along a normal of a projection plane of the image data, and an area determination from the image data to obtain the defined segment volumes.

In an example, the different positions are assigned to different pixels of an acquired image comprised in the image data.

In an example, the data processing unit 30 is further configured to normalize the corresponding dark-field signal value to a lung depth, the lung depth defined along a normal of a projection plane of the image data. In this way, dark field signals can be normalized to the lung depth.

In an example, the data processing unit 30 is further configured to determine the corresponding dark-field signal value of the segment volume on the basis of a lung depth determination, the lung depth defined along a normal of a projection plane of the image data. In this way, dark field signals can be normalized to the lung volume.

In an example, the data processing unit 30 is further configured to separate a left lung and a right lung to provide the segmented image data of the lung, wherein for each lung the first lung segment and the second lung segment is segmented. In other words, in a first step, the left and right lung may be separated from each other, and, in a second step, the left and/or right lung $L_{Left}$, $L_{Right}$ is segmented as described above.

Fig. 2 shows an example of a system 100 for processing dark field image data. The system 100 comprises at least one image acquisition device 110, and an apparatus 10 for presentation of dark field information as described with respect to Fig. 1 and any of the described examples or combination of examples. The at least one image acquisition device 110 is configured to provide the X-ray attenuation image, and to provide the dark field X-ray image. The data interface 20 is configured to output the X-ray attenuation image and/or the dark field X-ray image along with the quantitative measure.

In an example, the at least one image acquisition device 110 comprises a grating based dark field X-ray imaging device. In an example, the at least one image acquisition device 110 comprises an interferometer arrangement.

In an example, the at least one image acquisition device 110 comprises an X-ray imaging means. For example, the device may be a radiography system prepared for dark field imaging.

In an example, the at least one image acquisition device 110 may be configured to operate in a standard radiography mode, with transmitted intensities of radiation providing information on attenuation through the subject. In an example, the same image acquisition device 110 may be used to acquire the attenuation and dark field images. For example, the attenuation and dark field image data may be the output of the evaluation of the same acquisition data. This data may be multiple exposures (for example between 4 and 20) showing the image with different phase positions of one of the gratings. A full field system can be implemented with gratings as large as the detector.

In an example, the at least one image acquisition device 110 may be configured to generate an attenuation image, relating to the detection of intensity values of X-rays with and without the subject in the examination region. In an example, the at least one image acquisition device is configured to generate a dark field image, relating to the detection of fringe visibilities of the X-rays with and without the object in the examination region. In an example, the at least one image acquisition device is configured to generate any combination of these images. For example, the at least one image acquisition device may be configured to generate an attenuation image, and to generate a dark field image. In an example, an attenuation image and a dark field image may be generated at the same time. In an example, the summed spatial lungs volume information is the integral of an area of the lungs (derived from pixel area data) and the intensity of each pixel value.

Fig. 3 shows a method 200 for processing dark field image data. The method 200 comprises:
In a step, 210 the data processing unit 30 receives dark-field X-ray image data of a subject's lung.

In a step, 220, the data processing unit 30 segments the image data to provide segmented image data of the lung, wherein the segmented image data comprises at least a first lung segment and a second lung segment distinguishable from each other, and wherein the first lung segment and the second lung segment are segmented to have a defined volume.

In a step 230, the data processing unit 30 derives from the image data at least a first dark-field signal value assigned to the first lung segment and a second dark-field signal value assigned to the second lung segment.

In a step 240, the data processing unit 30 derives a quantitative measure by comparing the first dark-field signal value and the second dark-field signal value with each other, wherein the quantitative measure derived is output data for lung analysis.

In an example, the first lung segment and the second lung segment are segmented to have at least approximately equal volumes.

In an example, the step of comparing the first dark-field signal value and the second dark-field signal value comprises determining a ratio from the two values to derive the quantitative measure.

In an example, the ratio is used as an indicator of the presence of a disease or finding in the lungs of a subject.

In an example, the first lung segment is an upper lung field $L_{Upper}$ and the second lung segment is a lower lung field $L_{Left}$, the lung fields defined as upper and lower with respect to a longitudinal axis of a subject's body.

In an example the data processing unit 30 estimates a lung depth d, the lung depth defined along a normal of a projection plane of the image data, for a plurality of different positions within the corresponding lung segment.

In an example, the data processing unit 30 determines the corresponding segment volume V on the basis of a lung depth d determination, the lung depth L defined along a normal of a projection plane of the image data, and an area determination from the image data to obtain the at least approximately equal segment volumes.

In an example, the different positions p are assigned to different pixels of an acquired image recorded in the image data.

In an example, the data processing unit 30 normalizes the corresponding dark-field signal value to the lung depth d, wherein the lung depth is defined along a normal of a projection plane of the image data.

In an example, the data processing unit 30 determines the corresponding dark-field signal value DAX of the segment volume on the basis of a lung depth d determination, the lung depth d defined along a normal of a projection plane of the image data.

In an example, the data processing unit 30 separates a left lung $L_{Left}$ and a right lung $L_{Right}$ to provide the segmented image data of the lung, wherein for each lung $L_{Left}$, $L_{Right}$ the first lung segment and the second lung segment is segmented.

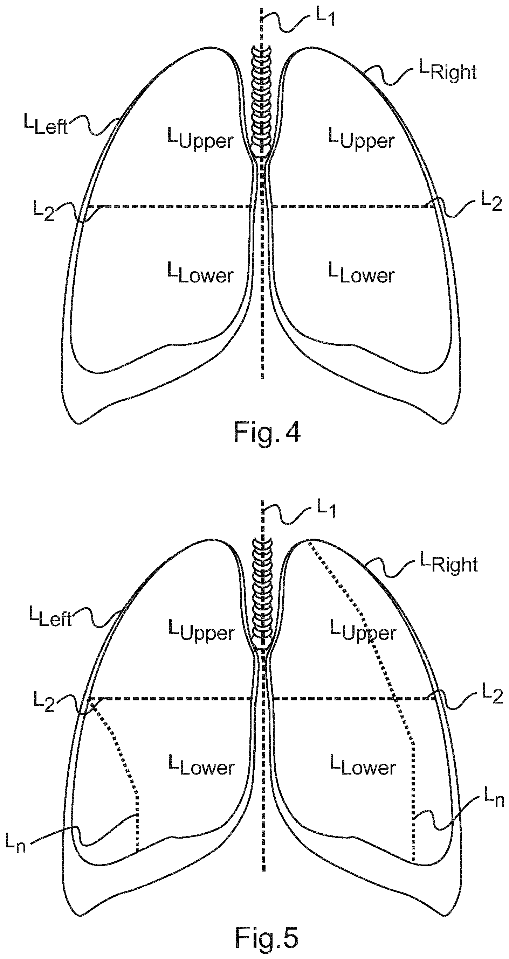

Fig. 4 shows an exemplary segmentation, e.g. separation, of a lung recorded in the image data, wherein the lung is segmented, e.g. separated, into the left lung $L_{Left}$ and right lung $L_{Right}$. Further, the left lung $L_{Left}$ and right lung $L_{Right}$ are segmented, e.g. separated, into its upper part $L_{Upper}$ and lower part $L_{Lower}$, wherein the volumes V of the respective upper part and lower part are defined or known, or preferably, are segmented to be equal to each other. In Fig. 4, the segmentation is indicated by dotted lines Li and $L_2$, where the dotted line Li indicates the segmentation of the left and right lung and the dotted line $L_2$ indicates the segmentation of the upper and lower lung $L_{Upper}$, $L_{Lower}$.

Fig. 5 shows another exemplary segmentation, e.g. separation, of a lung recorded in the image data, wherein at least one of the left lung $L_{Left}$ and the right lung $L_{Right}$ are segmented in more than the upper and lower part, namely into n parts, wherein this segmentation is indicated by dotted lines Ln.

In an exemplary embodiment, the data processing unit 30 segments, i.e. separates or divides, the lung recorded in the image data into multiple parts, e.g. four parts. First, the lung is separated into left lung and right lung. Then, at least one or both of the left lung and right is further segmented, i.e. separated or divided, into its upper and lower part, or into a medial and distal part, wherein each of the upper and lower lung part, or the medial and distal part, has a defined, i.e. known and/or determined, volume. In other words, the left and right lung is segmented, i.e. separated or divided, into parts with respect to the lung volume. In another exemplary embodiment, the left and right lung is segmented into more than two parts, e.g. into at least 3, or more, parts. The parts may be equally large with respect to the lung volume.

The right and left lung may be expressed as L, e.g. $L_{Left}$ and $L_{Right}$. The volume V of each lung part may be estimated based on the lung depth d(p) for each position p, e.g. image pixel, in L, e.g. in $L_{Left}$ and/or $L_{Right}$. The data processing unit 30 may then calculate a dark-field value for each position p, which dark-field value may be expressed as DAX(p). This dark-field value DAX (p) may be normalized to the lung depth d(p), which calculation of the normalized dark-field value NDAX may be expressed as

$$NDAX(p) = \frac{DAX(p)}{d(p)}$$

Then, a global normalized dark-field value NDAX may be calculated, which may be expressed as

$$NDAX = \frac{\sum_L DAX(p)}{\sum_L d(p)}$$

wherein $\sum_L DAX(p)$ gives a total lung volume by integrating d(p) over L. By restricting L to $L_1 < L$, sub volumes are given for every subarea Li, e.g. for the left and right lung $L_{Left}$, $L_{Right}$ only.

Based on this, the lung fields $L_{Left}$, $L_{Right}$ may be separated into sub fields with some given properties, such as the separation of left lung and right lung, the division of at least one side of the lung into equally large parts with respect to the lung volume by considering d(p).

By way of example, the quantitative measure may then comprise one or more of a difference between the left and right lung $L_{Left}$, $L_{Right}$ in the dark-field signal values DAX or NDAX, a standard deviation of the dark-field signal values DAX or NDAX over all segments and/or areas, etc., of the lung, a normalized standard deviation of the dark-field signal values DAX or NDAX (e.g. standard deviation or mean) over all segments and/or areas, etc., of the lung, and/or a ratio of the dark-field signal values DAX or NDAX of the lung segments. The quantitative measure may then be provided as output data for (further) lung analysis. Thereby, it has been found that both the (global) NDAX and the ratio of e.g. the upper and lower lung $L_U$, $L_L$ statistically separates subjects by their severity of emphysema. That is, emphysema subjects tend to have a higher $NDAX_{Upper}$-$NDAX_{Lower}$-ratio while subjects without emphysema typically have a lower one.

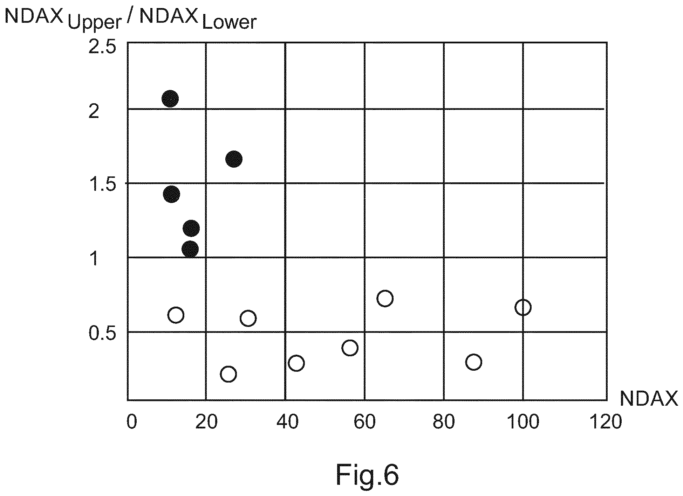

Fig. 6 shows a chart of a number of subjects, which were examined in a study, with different severity grades of emphysema and/or COPD given by spirometry. One dot represents one subject. The dots are positioned by the global NDAX, which is plotted on the x-axis, and by the $NDAX_{Upper}$-$NDAX_{Lower}$-ratio of the upper and lower lung segments $L_{Upper}$, $L_{Lower}$, which is plotted on the y-axis. In this chart, subjects having a higher $NDAX_{Upper}$-$NDAX_{Lower}$-ratio are indicated by a filled dot and subjects having a lower $NDAX_{Upper}$-$NDAX_{Lower}$-ratio are indicated by an unfilled dot. As can be seen in Fig. 4, the $NDAX_{Upper}$-$NDAX_{Lower}$-ratio determined as the quantitative measure provides means for distinguishing subjects with emphysema and/or COPD from subjects without emphysema and/or COPD.

Further details on x-ray dark field imaging can be found in the following papers: Pfeiffer, Franz, et al. "Hard-X-ray dark-field imaging using a grating interferometer." Nature materials 7.2 (2008), pages 134-137; and Yaroshenko, Andre, et al. "Pulmonary emphysema diagnosis with a preclinical small-animal x-ray dark-field scatter-contrast scanner." Radiology 269.2 (2013), pages 427-433.

In another exemplary embodiment, a computer program or computer program element is provided that is characterized by being configured to execute the method steps of the method according to one of the preceding embodiments, on an appropriate system.

The computer program element might therefore be stored on a data processing unit, which might also be part of an embodiment. This data processing unit may be configured to perform or induce performing of the steps of the method described above. Moreover, it may be configured to operate the components of the above described device and/or system. The computing unit can be configured to operate automatically and/or to execute the orders of a user. A computer program may be loaded into a working memory of a data processor. The data processor may thus be equipped to carry out the method according to one of the preceding embodiments.

Further, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and the foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

LIST OF REFERENCE SIGNS:

| 10 | apparatus |
|----|-----------|
| 20 | data interface |
| 30 | data processing unit |
| 100 | system |
| 110 | image acquisition device |
| 200 | method |
| 210 | method step |
| 220 | method step |
| 230 | method step |
| 240 | method step |

**Claims**

1. An apparatus (10) for processing dark-field X-ray image data information, comprising:

   a data interface (20); and
   a data processing unit (30);
   wherein the data interface (20) is configured to provide the data processing unit with dark-field X-ray image data,
   wherein the data processing unit (30) is configured to segment the image data to provide segmented image data of the lung, the segmented image data comprising at least a first lung segment and a second lung segment distinguishable therefrom, the first lung segment and the second lung segment being segmented to have a defined volume,
   wherein the data processing unit (30) is configured to derive, from the image data, at least a first dark-field signal value assigned to the first lung segment and a second dark-field signal value assigned to the second lung segment, and
   wherein the data processing unit (30) is configured to derive a quantitative measure by comparing the first dark-field signal value and the second dark-field signal value with each other, the quantitative measure being output data for lung analysis.

2. The apparatus according to claim 1, wherein the first lung segment and the second lung segment being segmented to have at least approximately equal volumes.

3. The apparatus according to claim 1 or 2, wherein comparing the first dark-field signal value and the second dark-field signal value comprises determining a ratio from the two values to derive the quantitative measure.

4. The apparatus according to claim 3, wherein the ratio is an indicator of the presence of a disease or finding in the lungs of a subject.

5. The apparatus according to any one of the preceding claims, wherein the first lung segment is an upper lung field and the second lung segment is a lower lung field, the lung fields defined as upper and lower with respect to a longitudinal axis of a subject's body.

6. The apparatus according to any one of the preceding claims, wherein the data processing unit (30) is further configured to estimate a lung depth, the lung depth defined along a normal of a projection plane of the image data, for a plurality of different positions within the corresponding lung segment.

7. The apparatus according to any one of the preceding claims, wherein the data processing unit (30) is further configured to determine the corresponding segment volume on the basis of a lung depth determination, the lung depth defined along a normal of a projection plane of the image data, and an area determination from the image data to obtain the at least approximately equal segment volumes.

8. The apparatus according to claim 5 or 6, wherein the different positions are assigned to different pixels of an acquired image comprised in the image data.

9. The apparatus according to any one of the preceding claims, wherein the data processing unit (30) is further configured to normalize the corresponding dark-field signal value to a lung depth, the lung depth defined along a normal of a projection plane of the image data.

10. The apparatus according to any one of the preceding claims, wherein the data processing unit (30) is further configured to determine the corresponding dark-field signal value of the segment volume on the basis of a lung depth determination, the lung depth defined along a normal of a projection plane of the image data.

11. The apparatus according to any one of the preceding claims, wherein the data processing unit (30) is further configured to separate a left lung and a right lung to provide the segmented image data of the lung, wherein for each lung the first lung segment and the second lung segment is segmented.

12. An X-ray imaging system for providing dark-field X-ray image data information, comprising:

an image acquisition device, configured to acquire X-ray image data of a subject; and
an apparatus according to any one of the preceding claims.

13. A method for providing dark-field X-ray image data information, comprising:

receiving, by a data processing unit (30), dark-field X-ray image data of a subject's lung;
segmenting, by the data processing unit (30), the image data to provide segmented image data of the lung, the segmented image data comprising at least a first lung segment and a second lung segment distinguishable therefrom, the first lung segment and the second lung segment being segmented to have a defined volume;
deriving, by the data processing unit (30), from the image data at least a first dark-field signal value assigned to the first lung segment and a second dark-field signal value assigned to the second lung segment; and
deriving, by the data processing unit (30), a quantitative measure by comparing the first dark-field signal value and the second dark-field signal value with each other, the quantitative measure being output data for lung analysis.

14. A computer program element, which when executed by a processor is configured to carry out the method of claim 13, and/or to control the apparatus (10) according to any one of claims 1 to 11, and/or to control the system (100) of claim 12.

15. A computer readable medium having stored the program element of claim 14.

Fig. 1

Fig. 2

Fig. 3

$L_1$

$L_{Right}$

$L_{Left}$

$L_{Upper}$

$L_{Upper}$

$L_2$

$L_2$

$L_{Lower}$

$L_{Lower}$

Fig. 4

$L_1$

$L_{Right}$

$L_{Left}$

$L_{Upper}$

$L_{Upper}$

$L_2$

$L_2$

$L_{Lower}$

$L_{Lower}$

$L_n$

$L_n$

Fig. 5

Fig.6

Europäisches
Patentamt

European
Patent Office

Office européen
des brevets

# EUROPEAN SEARCH REPORT

Application Number

EP 20 19 7184

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/271465 A1 (PROKSA ROLAND [DE] ET AL) 27 September 2018 (2018-09-27) * paragraphs [0103], [0091], [0094] * ----- | 1-15 | INV. A61B6/00 |
| A | R. GRADL ET AL: "Dynamic in vivo chest x-ray dark-field imaging in mice", IEEE TRANSACTIONS ON MEDICAL IMAGING, vol. 38, no. 2, 6 September 2018 (2018-09-06), pages 649-656, XP055557867, US ISSN: 0278-0062, DOI: 10.1109/TMI.2018.2868999 * page 653, left-hand column * ----- | 1-15 | |
| A | EP 3 622 891 A1 (KONINKLIJKE PHILIPS NV [NL]) 18 March 2020 (2020-03-18) * paragraph [0035] * ----- | 1-15 | |

TECHNICAL FIELDS
SEARCHED      (IPC)

A61B

The present search report has been drawn up for all claims

| Place of search | Date of completion of the search | Examiner |
|---|---|---|
| Munich | 3 March 2021 | Anscombe, Marcel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 19 7184

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2021

| Patent document cited in search report | | Publication date | Patent family member(s) | | Publication date |
|---|---|---|---|---|---|
| US 2018271465 | A1 | 27-09-2018 | CN 108348204 A | | 31-07-2018 |
| | | | EP 3355791 A1 | | 08-08-2018 |
| | | | JP 2018529443 A | | 11-10-2018 |
| | | | US 2018271465 A1 | | 27-09-2018 |
| | | | WO 2017055527 A1 | | 06-04-2017 |
| EP 3622891 | A1 | 18-03-2020 | EP 3622891 A1 | | 18-03-2020 |
| | | | WO 2020053345 A1 | | 19-03-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PFEIFFER, FRANZ et al.** Hard-X-ray dark-field imaging using a grating interferometer. *Nature materials,* 2008, vol. 7.2, 134-137

- **YAROSHENKO, ANDRE et al.** Pulmonary emphysema diagnosis with a preclinical small-animal x-ray dark-field scatter-contrast scanner. *Radiology,* 2013, vol. 269.2, 427-433